# NEUE EUROPÄISCHE PATENTSCHRIFT

(11) **EP 0 148 729 B2**
(45) Veröffentlichungstag und Bekanntmachung des Hinweises auf die Entscheidung über den Einspruch: **05.08.1998**
(45) Hinweis auf die Patenterteilung: 01.03.1989
(21) Anmeldenummer: 84810630.8
(22) Anmeldetag: 17.12.1984
(51) Int. Cl.: C07C 67/03, C07C 69/732, C07C 69/88

(54) **Verfahren zur Herstellung von sterisch gehinderten Hydroxyphenyl-carbonsäureestern**
Process for the preparation of sterically hindered hydroxyphenylcarboxylic-acid esters
Procédé de préparation d'esters d'acides hydroxyphénylcarboxyliques à encombrement stérique

(30) Priorität: 22.12.1983 CH 6857/83
(43) Veröffentlichungstag der Anmeldung: 17.07.1985
(73) Patentinhaber: Ciba Specialty Chemicals Holding Inc., 4057 Basel (CH)
(72) Erfinder: Orban, Ivan, Dr., CH-4052 Basel (CH)

(56) Entgegenhaltungen:
- EP-A- 0 102 920
- DE-A- 2 240 609
- DE-B- 1 543 644
- US-A- 3 644 482
- US-A- 4 093 587
- Patent Abstracts of Japan, vol. 1, no. 75 (1977), pp. 1475C77
- Chemical Abstracts, 87, 17, 1977, abstract 134692g

## Beschreibung

Die vorliegende Erfindung betrifft ein Verfahren zur Herstellung von sterisch gehinderten Hydroxyphenylcarbonsäureestern, bei welchem eine Umesterung ohne Lösungsmittel durch Behandlung mit einem Dialkylzinnoxid durchgeführt und die erhaltene Schmelze in einer Kurzzeit-Destillationsapparatur destilliert wird.

Umesterungsreaktionen zur Herstellung von sterisch gehinderten Hydroxyphenylcarbonsäureestern sind bekannt. So beschreiben z.B. die DE-AS 1 201 349 und die DE-OS 1 543 644 Umesterungsreaktionen dieser Art, bei welchen Alkalialkoholate als Katalysatoren eingesetzt werden. Umesterungsreaktionen der gleichen Art werden nach der DE-OS 2 150 327 mit Lithiumamid katalysiert. Bei all diesen Verfahren entstehen in mehr oder weniger kleinen Mengen Nebenprodukte (meistens Oxydationsprodukte von 2,6-Dialkylphenolen), die selbst in sehr geringen Mengen eine drastische Herabsetzung der Lagerstabilität des gewünschten Endproduktes verursachen. Die unumgängliche Entfernung dieser Nebenprodukte ist zeit-, arbeits- und energieaufwendig.

Die DE-OS 2 240 609 beschreibt in Beispiel 7 die Herstellung eines sterisch gehinderten Hydroxyphenylcarbonsäureesters durch Umesterung, wobei als Katalysator eine organische Titanverbindung eingesetzt wird.

Es wurde nun gefunden, dass die Durchführung der Umesterung ohne Lösungsmittel in Gegenwart katalytischer Mengen eines Dialkylzinnoxids mit anschliessender Destillation der erhaltenen Schmelze in einer Kurzzeit-Destillationsapparatur bei bestimmten Bedingungen überraschenderweise zu einer praktisch quantitativen Ausbeute an reinem Endprodukt führt, das, da keine störenden Nebenprodukte enthaltend, nicht zusätzlich gereinigt werden muss. Ein weiterer Vorteil dieses Verfahrens ist die lösungsmittelfreie Arbeitsweise.

Die vorliegende Erfindung betrifft demnach ein Verfahren zur Herstellung von Verbindungen der Formel I 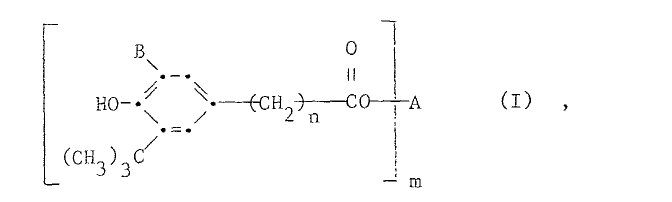 worin n die Zahlen 0 bis 2, m die Zahlen 1 bis 4, A einen von einem m-wertigen aliphatischen Alkohol abgeleiteten Rest mit 2 bis 18 Kohlenstoffatomen und B Methyl oder t-Butyl bedeuten, durch Umesterung von ca. m Molen eines Esters der Formel II 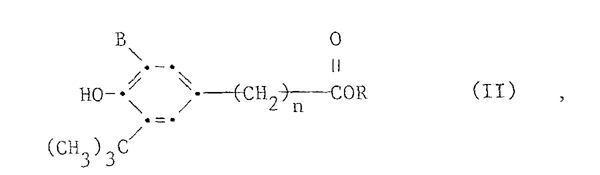 worin R Methyl oder Aethyl bedeutet, mit einem Alkohol der Formel (III)

A(̵OH)ₘ (III),

dadurch gekennzeichnet, dass
a) die Umesterung ohne Lösungsmittel durchgeführt wird,
b) die Umesterung in Gegenwart einer Zinnverbindung der Formel V 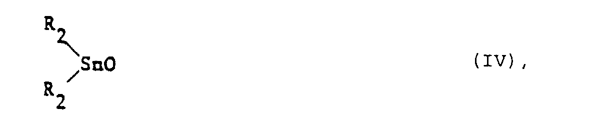 worin R₂ C₄-C₁₈-Alkyl bedeutet, als Katalysator in einer Menge zwischen 0,05 und 1,0 Mol%, bezogen auf den Ester der Formel II, durchgeführt und
c) die erhaltene Schmelze in einer Kurzzeit-Destillationsapparatur bei einem Druck zwischen 0,5 und 6 mbar und bei einer Temperatur zwischen 230 und 270°C destilliert und die anfallende Schmelze granuliert wird.

A ist als ein von einem m-wertigen aliphatischen Alkohol abgeleiteter Rest ein m-wertiger substituierter oder unsubstituierter aliphatischer Rest mit 2 bis 18 Kohlenstoffatomen.

Ist m 1, so handelt es sich bei A etwa um C₂-C₁₈ Alkyl, geradkettig oder verzweigt, wie z.B. Aethyl, n-Propyl, Isopropyl, n-Butyl, sec.-Butyl, tert.-Butyl, n-Pentyl, n-Hexyl, 2-Aethylhexyl, n-0ctyl, 1,1,3,3-Tetramethylbutyl, n-Decyl, n-Dodecyl, n-Hexadecyl oder n-0ctadecyl. Bevorzugt ist n-Ocatdecyl.

Ist m 2, so kann A beispielsweise C₂-C₁₈-Alkylen, bevorzugt C₂-C₆-Alkylen sein, wie Dimethylen, Trimethylen, Tetramethylen, Hexamethylen, 2,2-Dimethyl-trimethylen, Octamethylen, Nonamethylen, Decamethylen, Dodecamethylen oder Octadecamethylen. Die Alkylengruppe kann unterbrochen sein durch -O-, -S- oder -N(R)- wie in 2-Thiapropylen-1,3, 3-Thiapentylen-1,5, 4-Oxaheptamethylen, 3,6-Dioxaoctamethylen-1,8 oder 3,6-Diazaoctamethylen-1,8. Ist A unterbrochenes C₂-C₆-Alkylen, so ist es bevorzugt eine Gruppe 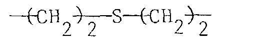 oder

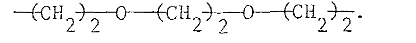

Ist m 3, so kann A ein trivalenter aliphatischer C₃H₅ bis C₇H₁₃ Kohlenwasserstoffrest sein, wie 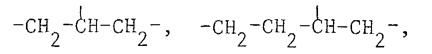 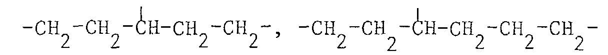 oder

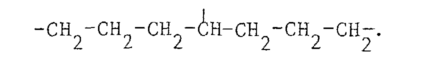

Falls m 4 ist, kann A eintetravalenter aliphatischer C₄H₆ bis C₁₀H₁₈ Kohlenwasserstoffrest sein, wie 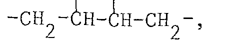 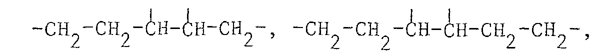 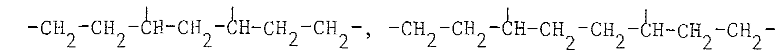 oder bevorzugt Pentaerythrityl.

R₂ in Formel V bedeutet als C₄-C₁₈-Alkyl z.B. n.-Butyl, n-Pentyl, n-Hexyl, n-Octyl, n-Decyl, n-Dodecyl, bevorzugt n-Butyl.

Der Katalysator wird bevorzugt in Mengen zwischen 0,1 und 0,3 Mol%, bezogen auf den Ester der Formel II, bei Temperaturen beispielsweise zwischen 110 und 220°C, besonders bevorzugt zwischen 120 und 200°C und z.B. bei einem Vakuum zwischen 250 und 3 mbar während 2 bis 5 Stunden eingesetzt.

Bei Verbindungen der Formel I, worin m>1 ist, ist es zweckmässig einen Ueberschuss von 10 - 30 Mol % an Ester der Formel II einzusetzen.

Geeignete Kurzzeit-Destillationsapparaturen sind z.B. Filmtruder, Fallfilmverdampfer und insbesondere Dünnschichtverdampfer. Die Destillation wird vorteilhaft kontinuierlich durchgeführt, jedoch ist eine diskontinuierliche Arbeitsweise auch möglich.

Die aus der Kurzzeit-Destillation erhaltene Schmelze braucht nur noch nach üblichen Methoden granuliert zu werden, z.B. auf dem Kühlband, um das fertige Endprodukt in praktisch reiner, freifliessender, staubfreier, für dessen spezifischen Einsatz bereiter Form zu erhalten.

Das erfindungsgemässe Verfahren ist besonders geeignet für die Herstellung von Verbindungen der Formel I durch Umesterung eines Esters der Formel II mit einem Alkohol der Formel III, worin in den Formeln I, II und III
- n: die Zahl 2 und m die Zahlen 1, 2 oder 4,
- A: bei m = 1 C₂-C₁₈-Alkyl,
bei m = 2 C₂-C₆-Alkylen, eine Gruppe 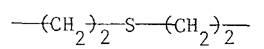 oder 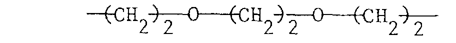 und bei m = 4 Pentaerythrityl und
- R: Methyl bedeuten, insbesondere für die Herstellung von Pentaerythrityl-tetrakis[3-(3,5-di-tert.-butyl-4-hydroxyphenyl)-propionat] durch Umesterung von Methyl-3-(3,5-di-tert.-butyl-4-hydroxyphenyl)-propionat mit Pentaerythrit.

Bevorzugt wird die erfindungsgemässe Umesterung bei Katalysatormengen zwischen 0,1 und 0,3 Mol%, bezogen auf die Verbindung der Formel II, bei Temperaturen zwischen 110 und 220°C und bei einem Vakuum zwischen 250 und 3 mbar durchgeführt.

Die Destillation der Schmelze (Stufe c) wird vorzugsweise bei einem Druck zwischen 1 und 3 mbar und bei einer Temperatur zwischen 240 und 260°C ausgeführt.

Bei den Estern der Formel II, den Alkoholen der Formel III und den Katalysatoren handelt es sich um bekannte Substanzen. Sollten einzelne dieser Substanzen neu sein, so können sie nach allgemein bekannten Verfahren hergestellt werden.

Die Verbindungen der Formel I sind wertvolle Stabilisatoren für organische Materialien, die der Zersetzung unterworfen sind, zum Beispiel für synthetische organische Polymere, tierische und pflanzliche Oele, Kohlenwasserstoffe, Schmiermittel und dergleichen.

Die folgenden Beispiele erläutern die Erfindung.

### Beispiel 1:

a) In einem Kolben, welcher mit einem auf 80°C beheizten Dephlegmator versehen ist, werden 1533 g (5,25 Mol) Methyl-3-(3,5-di-tert.- butyl-4-hydroxyphenyl)-propionat und 143 g (1,05 Mol) Pentaerythrit vorgelegt und auf 80°C erwärmt. Es werden dann 2,1 g (0,0084 Mol) Dibutylzinnoxid zugegeben und unter Stickstoff bei einem Vakuum von 250 mbar auf 180°C erhitzt. Das entstehende Methanol wird dabei in die Vorlage abdestilliert. Die Destillation wird bei 180°C und 250 mbar während weiteren 45 Minuten fortgesetzt und anschliessend das Vakuum innerhalb einer Stunde sukzessive auf ca. 4 mbar verbessert. Zum Schluss wird für weitere 30 Minuten bei 180°C und 3 bis 4 mbar gerührt. Die erhaltene leicht gelbliche Schmelze wird auf 80 - 100°C abgekühlt und bis zu ihrer Weiterverarbeitung unter Stickstoff aufbewahrt.
b) Die unter a) erhaltene Schmelze wird in Portionen von ca. 300 ml durch einen Dünnschichtverdampfer bei einem Vakuum von 1 - 3 mbar und einer Temperatur im Verdampferteil von 260°C gelassen. Die Temperatur im Zulaufgefäss und im Auffanggefäss ist 150°C. Der Durchsatz ist von ca. 1000 g in der Stunde bei einer Rotordrehzahl von ca. 50 Upm. Dabei werden die Schmelze des Endprodukts im Auffanggefäss und das überschüssige Methyl-3-(3,5-di-tert.-butyl-4-hydroxyphenyl)-propionat mit Spuren von Methanol in der Destillationsvorlage aufgefangen.

Jeweils nach Ende eines Durchlaufes wird die Apparatur mit Stickstoff entlastet und die Produkteschmelze aus dem Auffanggefäss auf ein Blech zur Erstarrung gelassen. Die erstarrte Schmelze wird anschliessend zerkleinert. Unter Betriebsbedingungen wird die Schmelze direkt zur Granulierung geführt.
Man erhält 1232 g (99,7 % d.Th.) Pentaerythrityl-tetrakis-[3-(3,5-di-tert.-butyl-4-hydroxyphenyl)-propionat] mit Smp. 58 - 67°C.

### Beispiel 2:

a) In einem Kolben werden 1051 g (3,6 Mol) Methyl-3-(3,5-di-tert.-butyl-4-hydroxyphenyl)-propionat und 1001 g (3,7 Mol) Stearylalkohol vorgelegt und auf 80°C erwärmt. Es werden dann 1,8 g (0,0072 Mol) Dibutylzinnoxid zugegeben und unter Stickstoff bei einem Vakuum von 250 mbar auf 140°C erhitzt. Das entstehende Methanol wird dabei in die Vorlage abdestilliert. Nun wird bei 140°C das Vakuum innerhalb einer Stunde sukzessive auf ca. 4 mbar verbessert. Zum Schluss wird für weitere 30 Minuten bei 140°C und 3-4 mbar gerührt. Die erhaltene leicht gelbliche Schmelze wird auf 80 - 100°C abgekühlt und bis zu ihrer Weiterverarbeitung unter Stickstoff aufbewahrt.
b) Die unter a) erhaltene Schmelze wird genau wie unter Beispiel 1b) beschrieben weiterverarbeitet.

Man erhält 1905 g (99.8 % d. Th) Stearyl-3-(3,5-di-tert.-butyl-4-hydroxyphenyl)-propionat mit Smp. 50 - 51°C.

Beispiel 3: Das Verfahren des Beispiels 2 wird wiederholt mit der einzigen Ausnahme, dass statt 1001 g nur 972 g (3,6 Mol) Stearylalkohol eingesetzt werden.

Man erhält 1901 g (99,6% d.Th) Stearyl-3-(3,5-di-tert.-butyl-4-hydroxyphenyl)-propionat mit Smp. 50 - 51°C.

Beispiel 4: Das Verfahren des Beispiels 1 wird wiederholt mit der einzigen Ausnahme, dass die Umesterung bei 190, statt bei 180°C durchgeführt wird. Man erhält das Produkt von Beispiel 1 mit gleichem Schmelzpunkt und gleicher Ausbeute.

## Patentansprüche

1. Verfahren zur Herstellung von Verbindungen der Formel I 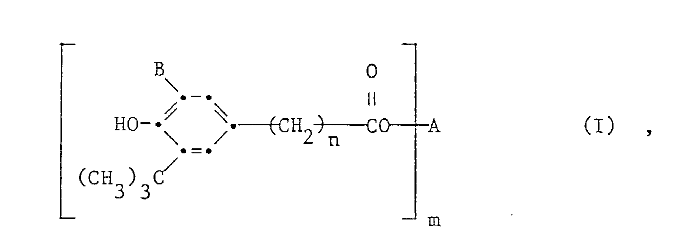 worin n die Zahlen 0 bis 2, m die Zahlen 1 bis 4, A einen von einem m-wertigen aliphatischen Alkohol abgeleiteten Rest mit 2 bis 18 Kohlenstoffatomen und B Methyl oder t-Butyl bedeuten, durch Umesterung von ca. m Molen oder, wenn m grösser als 1 ist, zweckmässig in einem Ueberschuss von 10-30 Mol% eines Esters der Formel II 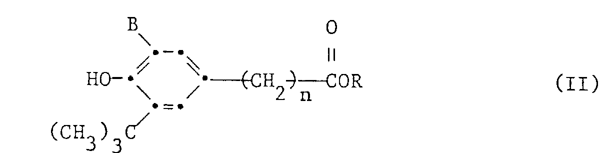 worin R Methyl oder Aethyl bedeutet, mit einem Alkohol der Formel (III)
A(̵OH)ₘ (III)
dadurch gekennzeichnet, dass
a) die Umesterung ohne Lösungsmittel durchgeführt wird,
b) die Umesterung in Gegenwart einer Zinnverbindung der Formel IV 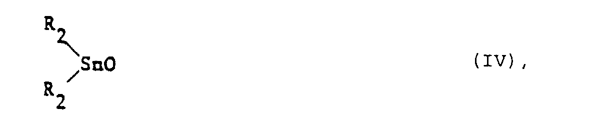 worin R₂ C₄-C₁₈-Alkyl bedeutet, als Katalysator in einer Menge zwischen 0,05 und 1,0 Mol%, bezogen auf den Ester der Formel II, durchgeführt und
c) die erhaltene Schmelze in einer Kurzzeit-Destillationsapparatur bei einem Druck zwischen 0,5 und 6 mbar und bei einer Temperatur zwischen 230 und 270°C destilliert und die anfallende Schmelze granuliert wird.

2. Verfahren gemäss Anspruch 1, dadurch gekennzeichnet, dass die Verbindung der Formel IV Dibutylzinnoxid ist.

3. Verfahren gemäss Anspruch 1 zur Herstellung von Verbindungen der Formel I durch Umesterung eines Esters der Formel II mit einem Alkohol der Formel III, wobei in den Formeln I, II und III
n die Zahl 2 und m die Zahlen 1, 2 oder 4,
A bei m = 1 C₂-C₁₈-Alkyl,
bei m = 2 C₂-C₆-Alkylen, eine Gruppe 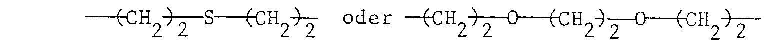 und
bei m = 4 Pentaerythrityl und
R Methyl bedeuten.

4. Verfahren gemäss Anspruch 1, dadurch gekennzeichnet, dass bei der Umesterung der Katalysator in Mengen zwischen 0,1 und 0,3 Mol % , bezogen auf den Ester der Formel II, bei Temperaturen zwischen 110 und 220°C und bei einem Vakuum zwischen 250 und 3 mbar eingesetzt wird.

5. Verfahren gemäss Anspruch 1, dadurch gekennzeichnet, dass die Destillation der Schmelze in der Kurzzeit-Destillationsapparatur bei einem Druck zwischen 1 und 3 mbar und bei einer Temperatur zwischen 240 und 260°C durchgeführt wird.

6. Verfahren gemäss Anspruch 1 zur Herstellung von Pentaerythrityltetrakis[3-(3,5-di-tert.-butyl-4-hydroxyphenyl)-propionat] durch Umesterung von Methyl-3-(3,5-di-tert.-butyl-4-hydroxyphenyl)-propionat mit Pentaerythrit.

## Claims

1. A process for the preparation of compounds of the formula I 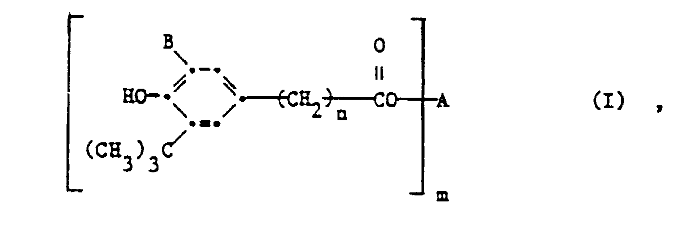 in which n is the numbers 0 to 2, m is the numbers 1 to 4, A is a radical which is derived from an m-hydric aliphatic alcohol and has 2 to 18 carbon atoms and B is methyl or t-butyl, by transesterifying about m moles or, if m is greater than 1, advantageously an excess of 10-30 mol % of an ester of the formula II 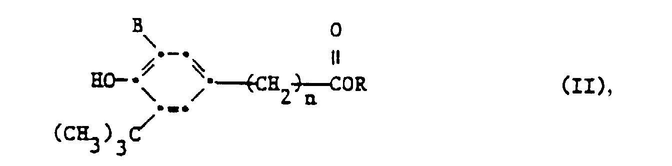 in which R is methyl or ethyl, with an alcohol of the formula III
A(̵OH)ₘ (III),
which comprises a) carrying out the transesterification in the absence of a solvent, b) carrying out the transesterification in the presence of a tin compound of the formula IV, 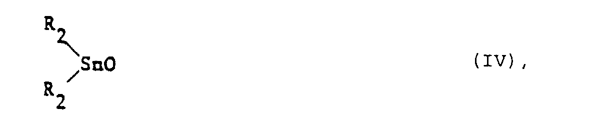 in which R₂ is C₄-C₁₈ alkyl, as catalyst, in an amount between 0.05 and 1.0 mol %, based on the ester of the formula II, and c) distilling the resulting melt in a flash distillation apparatus under a pressure of between 0.5 and 6 mbar and at a temperature between 230 and 270°C, and granulating the resulting melt.

2. A process according to claim 1, wherein the compound of the formula IV is dibutyltin oxide.

3. A process according to claim 1 for the preparation of compounds of the formula I by transesterifying an ester of the formula II with an alcohol of the formula III, where in the formulae I, II and III n is the number 2 and m is the numbers 1, 2 or 4, A is C₂-C₁₈alkyl if m = 1, C₂-C₆alkylene, a group 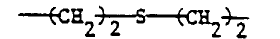 or 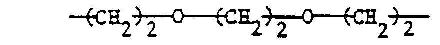 if m = 2, and pentaerythrityl if m = 4, and R is methyl.

4. A process according to claim 1, wherein the catalyst in the transesterification is employed in amounts between 0.1 and 0.3 mol %, based on the ester of the formula II, at temperatures between 110 and 220°C and under a vacuum of between 250 and 3 mbar.

5. A process according to claim 1, wherein the distillation of the melt in the flash distillation apparatus is carried out under a pressure of between 1 and 3 mbar and at a temperature between 240 and 260°C.

6. A process according to claim 1 for the preparation of pentaerythrityl tetrakis[3-(3,5-di-tert-butyl-4-hydroxyphenyl)propionate] by transesterifying methyl 3-(3,5-di-tert-butyl-4-hydroxyphenyl)propionate with pentaerythritol.

## Revendications

1. Procédé de préparation de composés de formule 1 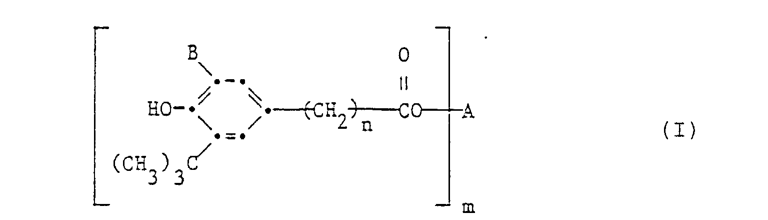 dans laquelle n va de 0 à 2, m va de 1 à 4, A représente un reste avec 2 à 18 atomes de carbone dérivant d'un alcool aliphatique à fonctionnalité égale à m, et B représente un groupe méthyle ou t-butyle,
par transestérification d'environ m moles, ou lorsque m est supérieur à 1, de façon appropriée dans un excès de 10 à 30 % en poids d'un ester de formule II 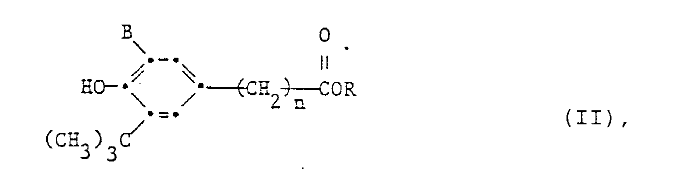 dans laquelle R représente un groupe méthyle ou alkyle, avec un alcool de formule III
A-(-OH)ₘ (III)
caractérisé en ce que
a) l'on met en oeuvre la transestérification sans solvant,
b) l'on met en oeuvre la transestérification en présence d'un composé d'étain de formule IV 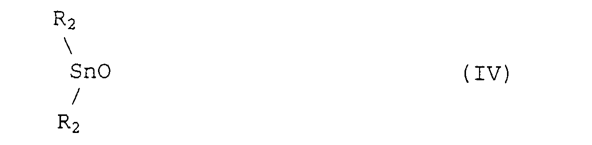 dans laquelle R₂ représente un groupe alkyle en C₄-C₁₈, en tant que catalyseur, dans une quantité comprise entre 0,05 et 1,0 % en moles, par rapport à l'ester de formule II, et
c) on granule la matière fondue obtenue dans un appareil de distillation à temps court, sous une pression comprise entre 0,5 et 6 mbar et une température comprise entre 230 et 270 °C et on granule la masse fondue obtenue.

2. Procédé selon la revendication 1, caractérisé en ce que le composé de formule IV est l'oxyde de dibutylétain.

3. Procédé selon la revendication 1 pour la préparation de composés de formule I par transestérification d'un ester de formule II avec un alcool de formule III, dans les formules I, II et III
n vaut 2 et m vaut 1, 2 ou 4,
A représente
pour m = 1, un groupe alkyle en C₂-C₁₈,
pour m = 2, un groupe alkylène en C₂-C₆, un groupe
-(-CH₂-)₂-S-(-CH₂-)₂- ou
-(-CH₂-)₂-O-(-CH₂-)₂-O-(-CH₂-)₂- et
pour m = 4, le pentaérythrityle, et
R représente un groupe méthyle.

4. Procédé selon la revendication 1, caractérisé en ce que l'on utilise le catalyseur dans la transestérification dans une quantité comprise entre 0,1 et 0,3 % en moles par rapport à l'ester de formule II, à une température comprise entre 110 et 220 °C et sous un vide compris entre 250 et 3 mbar.

5. Procédé selon la revendication 1, caractérisé en ce que l'on met en oeuvre la distillation de la matière fondue dans un appareil de distillation à temps court, sous une pression comprise entre 1 et 3 mbar et à une température comprise entre 240 et 260 °C.

6. Procédé selon la revendication 1 pour la préparation du tétrakis-[3-(3,5-di-tert.-butyl-4-hydroxyphényl)-propionate de pentaérythrityle par transestérification du 3-(3,5-di-tert.-butyl-4-hydroxyphényl)-propionate de méthyle avec le pentaérythritol.
